# EUROPÄISCHE PATENTSCHRIFT

(11) **EP 0 535 178 B1**
(45) Veröffentlichungstag und Bekanntmachung des Hinweises auf die Patenterteilung: **18.11.1998**
(21) Anmeldenummer: 92905314.8
(22) Anmeldetag: 26.02.1992
(51) Int. Cl.: A61K 9/36, A61K 9/28

(54) **HALOGENKOHLENWASSERSTOFFFREIE RETARDLACKLÖSUNG FÜR PHARMAZEUTISCHE ZUBEREITUNGEN**
HALOHYDROCARBON-FREE TIMED-RELEASE COATING SOLUTION FOR PHARMACEUTICAL PREPARATIONS
SOLUTION D'ENROBAGE RETARDATEUR DE LIBERATION, EXEMPTE D'HYDROCARBURES HALOGENES, POUR PREPARATIONS PHARMACEUTIQUES

(30) Priorität: 27.02.1991 DE 4106090
(43) Veröffentlichungstag der Anmeldung: 07.04.1993
(73) Patentinhaber: BOEHRINGER INGELHEIM INTERNATIONAL GmbH, 55218 Ingelheim am Rhein (DE); Boehringer Ingelheim Pharma KG, 55216 Ingelheim am Rhein (DE)
(72) Erfinder: WALZ, Michael, D-6530 Bingen/Rhein (DE)
(74) Vertreter: Laudien, Dieter, Dr.
(86) Internationale Anmeldenummer: EP9200395
(87) Internationale Veröffentlichungsnummer: WO9215287

(56) Entgegenhaltungen:
- FR-A- 2 430 766
- Chemical Abstracts, Band 85, Nr. 8, 23. August 1976, (Columbus, Ohio, US) T.A. Groshovyi et al.: "Development of an optimum film-forming composition based on ethylcellulose for protective coating of tablets in a pseudoliquified", siehe Seite 335, Spalte 1, Zusammenfassung 51697v, & Farm. Zh. (Kiev) 1976, 31(2), 65-8
- Acta Pharmaceutica Technologica, Band 27, Nr. 3, 1981, (Stuttgart, DE) B.C. Lippold et al.: "Diffusion von Theophyllin durch isolierte zuschlaghaltige Ethylcellulose-Membranen", Seiten 169-179, siehe Seite 173, Tabelle 1

## Beschreibung

Die Erfindung betrifft eine Retardlacklösung für pharmazeutische Zubereitungen, die sich dadurch auszeichnet, daß sie frei von halogenhaltigen Kohlenwasserstoffen ist.

In Hinblick auf die Steuerung der Wirkstofffreigabe bei festen Arzneiformen unterscheidet man verschiedene Ansätze und Systeme mit unterschiedlichen Eigenschaften und Freigabecharakteristika (Matrixsysteme, Hüllensysteme etc. A.N. Martin, J. Swarbrick, A. Cammarata; H. Stricker (Hrsg.), Physikalische Pharmazie, 3. Aufl., Stuttgart: Wissenschaftliche Verlagsgesellschaft, 1987).
Für die Erzielung einer konstanten Wirkstoff-Freigabe hat sich mehr und mehr das Prinzip des Überziehens wirkstoffhaltiger Formlinge mit semipermeablen Diffusionshüllen durchgesetzt. Oft bestehen derartige Hüllen aus einer unlöslichen Komponente wie z.B. Ethylcellulose und einer hierin eingelagerten löslichen Komponente wie z.B. Polyethylenglykol. Eine typische Rezeptur für derartige Retardlacke besteht beispielsweise aus Ethylcellulose und Polyethylenglykol als Feststoffe und Ethanol und Dichlormethan als Lösungsmittel. Halogenkohlenwasserstoffe, insbesondere Dichlormethan, zeichnen sich durch ein hervorragendes Lösungsvermögen für viele pharmazeutische Hilfsstoffe aus und wurden bislang gerne als Lösungsmittel zur Herstellung von solchen Überzügen auf pharmazeutischen Zubereitungen, wie z.B. Tabletten, Pellets und Granulaten verwandt.

Allerdings ist die Verwendung von halogenierten Krohlenwasserstoffen aus umweltschutztechnischen und toyikologischen Gründen in Frage zu stellen, so daß ein Verzicht auf diese Stoffe wünschenswert ist.

Ein einfacher Austausch der halogenhaltigen Retardlacklösungen gegen wässerige Dispersionssysteme, die seit einiger Zeit auf dem Markt verfügbar sind, ist jedoch nicht möglich. Die Filmhüllen aus diesen Systemen zeigen meist ein völlig anderes Freigabeverhalten. Darüber hinaus ergeben sich sehr oft Probleme mit der Konstanz des Freigabeverhaltens bzw. Stabilitätsprobleme aufgrund von Wechselwirkungen zwischen Wirkstoff und den technologisch notwendigen Hilfsstoffen in diesen Zubereitungen. Die Verwendung halogenfreier organischer Lösungsmittelsysteme scheiterte bislang oft an den schlechten Löseeigenschaften der alternativen Lösungsmittel für die Lackkomponenten wie z.B. Ethylcellulose und Polyethylenglykol und vor allem an dem anderen Freigabeverhalten der Filmüberzüge aus solchen Retardlacksystemen. Die Struktur der Lackhülle und damit das Freigabeverhalten wird auch hier wesentlich vom verwendeten Lösungsmittel mitbestimmt.

So wurden bei ersten Versuchen mit verschiedenen Lösungsmittelgemischen teilweise entweder ein ungenügender Retardierungseffekt oder aber eine übermäßige Verzögerung der Wirkstofffreigabe beobachtet. Viele Lösungsmittelsysteme waren wegen der schlechten Löseeigenschaften für die Filmbildner nicht verarbeitbar.

Überraschenderweise wurde gefunden, darf bei Verwendung von bestimmten Lösungsmittelsystemen mit einem genau definierten, relativ geringen Wasseranteil gut verarbeitbare Retardlacklösungen hergestellt werden können, die Diffusionshüllen mit einem Freigabeverhalten ergeben, die denen aus halogenkohlenstoffhaltigen Retardlacklösungen hergestellten vergleichbar sind.

Erfindungsgemäß wird eine Retardlacklösung vorgeschlagen, die Ethylcellulose als wasserunlöslicher Filmbildner, Polyethylenglycol als wasserlöslicher Porenbildner und als Lösungsmittel einen oder mehrere niedrig siedende(n) Alkohol(e) oder Mischungen aus jeweils einem oder mehreren niedrig siedenden Alkohol(en) und Keton(en) enthält, dadurch gekennzeichnet, daß das Lösungsmittel einen Wasserzusatz von 0,5 - 10 Gew.-% enthält und daß der Feststoffanteil der Retardlacklösung selbst 50 - 70 Gew.-% Ethylcellulose und 30 - 50 Gew.-% Polyethylenglycol enthält.

Die erfindungsgemäße Retardlacklösung ist zur Herstellung von Diffussionshüllen zur Freigabesteuerung pharmazeutischer Wirkstoffe durch Versprühen (unter geeigneten Bedingungen) auf Formlinge (Preßlinge, Pellets und sonstige pharmazeutische Formlinge) bestimmt.

Die geeigneten Bedingungen betreffen u. a.
- Auflösungs- und Ausdehnungsverhalten der Formlinge
- technische Faktoren des Überziehgerätes (Art des Gerätes, Größe, Düse, Sprühabstand etc.)
- Klimabedingungen bei der Sprühung
- Prozeßführung bei der Sprühung (Sprühgeschwindigkeit, Trocknungsmodus etc.).

Die Bedingungen entsprechen im wesentlichen denen der bekannten halogenwasserstoffhaltigen Lösungen und sind dem Fachmann bekannt.

Die solchermaßen überzogenen Formlinge weisen vergleichbare Eigenschaften auf, speziell hinsichtlich dei Wirkstofffreisetzung, wie die mit den üblichen halogenhaltigen/dichlormethanhaltigen Lacklösungen überzogenen Formlinge.

Als wasserunlöslicher Filmbildner wird erfindungsgemäß Ethylcellulose pharmazeutischer Qualität, z.B. mit einem Ethoxylgehalt von ca. 45 - 50 %, vorzugsweise ca. 48 - 49,5 % (z.B. Fa. Hercules Type N) und einer Viskosität (5 %-ige Lösung in Toluol/Ethanol 80 : 20) von 8 - 24, cps vorzugsweise 12 - 16 cps. (z.B. Typ N 14 der Firma Hercules) verwendet; als wasserlösliches Polymer wird Polyethylenglycol verwendet. Das Molekulargewicht kann zwischen 4000 und 10 000, vorzugsweise zwischen 4000 und 8000 (z.B. Polyethylenglycol Typ 6000 der Firma Hoechst) betragen.

Die Konzentration der Feststoffe im Lösungsmittel beträgt 2 - 15 Gew.-%, vorzugsweise 5 - 10 Gew.-%. Vom Feststoff beträgt der Anteil an Ethylcellulose 50 - 70 Gew.-% und der Anteil an Polyethylenglycol 30 - 50 Gew.-%.

Geeignete Lösungsmittel sind niedrig siedende Alkohole, wie z.B. Methanol, Ethanol, Propanol, Isopropanol, 1-Butanol, 2-Butanol, wobei Ethanol und Isopropanol bevorzugt sind.

Der Wasserzusatz beträgt 0.5 - 10 % jeweils berechnet auf den wasserfreien Alkohol.

Geeignet sind ferner Mischungen aus niedrigsiedenden Alkoholen und Ketonen, vorzugsweise Ethanol/Aceton oder Isopropanol/Aceton. Ein weiteres geeignetes Keton ist Methylethylketon.

Das Verhältnis von Alkohol zu Keton kann in einem Verhältnis von 10 : 90 bis 90 : 10, vorzugsweise 40 : 60 bis 60 : 40 variieren, wobei der Wasserzusatz zwischen 0.5 - 10 %, vorzugsweise 1 - 5 % liegt.

Eine bevorzugte Retardlacklösung der Erfindung enthält Ethanol mit 4 - 10 % Wasser oder Ethanol/Aceton 1 : 1 mit 1 - 5 % Wasser als Lösungsmittel, 5 - 10 % Feststoff bestehend aus 50 - 70 % Ethylcellulose und 30 - 50 % Polyethylenglycol.

Die nachfolgenden Beispiele sollen die Erfindung näher erläutern, ohne sie jedoch auf die ausgeführten Beispiele einzuschränken.

### Beispiel 1

### Tabletten

| Parameter | |
|---|---|
| Wirkstoff | Bunitrolol |
| Form | rund, gewölbt, Wölbungsradius 5.5 mm |
| Maße | Durchmesser 5 mm, Dicke ca. 2.5 mm |
| Masse | 50 mg |

| Hüllenbestandteile | | |
|---|---|---|
| Bezeichnung | Ethylcellulose | Polyethylenglykol |
| Typ | N 14 | 6000 |
| Hersteller | Hercules | Hoechst |
| Feststoffanteil | 60 % | 40 % |

| Lösungsmittel | | | |
|---|---|---|---|
| Bezeichnung | Aceton | Ethanol | Wasser |
| Qualität | technische Ware | Ethanol abs. vergällt mit Methylethylketon | gereinigtes Wasser |
| Zusammensetzung Lösunsmittelsystem | 49,5 % | 49,5 % | 1 % |

| Retardlacklösungen Parameter | |
|---|---|
| Feststoffkonzentration | 10 % |
| Herstellung | - Einwiegen des Lösungsmittelgemisches |
| | - Lösen von Polyethylenglykol 6000 (unter Erwärmung auf ca. 40°C) |
| | - Einrühren von Ethylcellulose N 14 (nach Abkühlen auf unter 30°C) |
| | - Durchmischen der Lösung |

| Filmsprühung Parameter | |
|---|---|
| Ansatzgröße Tabletten | 10 kg = ca. 200.000 Tabletten |
| Lackmenge je Tablette | 6 mg |
| Überziehgerät | Accela-Cota 24 (Fa. Manesty, Liverpool) |
| Sprühpistole | Walther Pilot WA XV |
| | Düse: 1.3 mm |
| Sprühluftüberdruck | 1.5 bar |
| Zuluft | ca. 51°C |
| Abluft | ca. 31°C |
| Gutstemperatur | ca. 32°C |

| Trocknung der Filmtabletten Parameter | |
|---|---|
| Trocknungsgerät | Accela-Cota |
| Zuluft | 40°C |

### Beispiel 2

### Tabletten

| Parameter | |
|---|---|
| Wirkstoff | Bunitrolol |
| Form | rund, gewölbt, Wölbungsradius 5.5 mm |
| Maße | Durchmesser 5 mm, Dicke ca. 2.5 mm |
| Masse | 50 mg |

| Hüllenbestandteile | | |
|---|---|---|
| Bezeichnung | Ethylcellulose | Polyethylenglykol |
| Typ | N 14 | 6000 |
| Hersteller | Hercules | Hoechst |
| Feststoffanteil | 60 % | 40 % |

| Lösungsmittel | | | |
|---|---|---|---|
| Bezeichnung | Aceton | Ethanol | Wasser |
| Qualität | technische Ware | Ethanol abs. vergällt mit Methylethylketon | gereinigtes Wasser |
| Zusammen setzung | --- | 96 % | 4 % |

### Lösungsmittelsystem

Die weitere Herstellung erfolgt wie in Beispiel 1 angegeben.

## Patentansprüche

1. Retardlacklösung für pharmazeutische Zubereitungen enthaltend Ethylcellulose als wasserunlöslicher Filmbildner, Polyethylenglycol als wasserlöslicher Porenbildner und als Lösungsmittel einen oder mehrere niedrig siedende(n) Alkohol(e) oder Mischungen aus jeweils einem oder mehreren niedrig siedenden Alkohol(en) und Keton(en), dadurch gekennzeichnet, daß das Lösungsmittel einen Wasserzusatz von 0,5 - 10 Gew.-% enthält und daß der Feststoffanteil der Retardlacklösung selbst 50 - 70 Gew.-% Ethylcellulose mit einem Ethoxylgehalt von ca. 45-50% und einer Viskosität (5%-ige Lösung in Toluol/Ethanol 80.20) von 8-24 cps sowie 30-50 Gew.-% Polyethylenglycol mit einem Molekulargewicht zwischen 4000 und 10000 enthält.

2. Retardlacklösung gemäß Anspruch 1, dadurch gekennzeichnet, daß der Feststoffanteil 2 - 15 Gew.-% beträgt.

3. Retardlacklösung gemäß einem der Ansprüche 1 oder 2, dadurch gekennzeichnet, daß das Lösungsmittel wasserfreies Ethanol, Isopropanol mit einem Wasserzusatz von 4 - 10 Gew.-% oder ein Gemisch aus wasserfreiem Ethanol/Aceton oder Isopropanol/Aceton im Verhältnis 1 : 1 mit einem Wasserzusatz von 1 -5 Gew.-% ist.

4. Retardlacklösung gemäß einem der Ansprüche 1, 2 oder 3, dadurch gekennzeichnet, daß Polyethylenglycol 6000 verwendet wird

5. Retardlacklösung gemäß einem der Ansprüche 1, 2, 3 oder 4, dadurch gekennzeichnet, daß Ethylcellulose mit einem Ethoxylgehalt von ca. 48-49,5% und einer Viskosität (5%-ige Lösung in Toluol/Ethanol 80:20) von 12-16 cps verwendet wird.

6. Verwendung einer Retardlacklösung gemäß einem der Ansprüche 1 bis 5 zur Herstellung von Diffusionshüllen auf pharmazeutischen Zubereitungen zur Freigabesteuerung pharmazeutischer Wirkstoffe.

## Claims

1. Delayed-release coating solution for pharmaceutical preparations containing ethylcellulose as water-insoluble film forming agent, polyethyleneglycol as water-soluble pore forming agent and as solvent one or more low boiling alcohols or mixtures of one or more low boiling alcohols and ketones, characterised in that the solvent has water added thereto in an amount from 0.5-10% by weight and the solid fraction of the delayed-release solution itself contains 50-70% by weight of ethylcellulose with an ethoxyl content of about 45-50% and a viscosity (5% solution in toluene/ethanol 80:20) of 8-24 cps and 30-50% by weight of polyethyleneglycol with a molecular weight of between 4,000 and 10,000.

2. Delayed-release coating solution according to claim 1, characterised in that the solids content is from 2-15% by weight.

3. Delayed-release coating solution according to one of claims 1 and 2, characterised in that the solvent is anhydrous ethanol, isopropanol containing 4-10% by weight added water or a mixture of anhydrous ethanol/ acetone or isopropanol/acetone in the ratio 1:1 with added water in an amount from 1-5% by weight.

4. Delayed-release coating solution according to one of claims 1, 2 and 3, characterised in that polyethyleneglycol 6000 is used.

5. Delayed-release coating solution according to one of claims 1, 2, 3 or 4, characterised in that ethylcellulose with an ethoxyl content of about 48-49.5% and a viscosity (5% solution in toluene/ethanol 80:20) of 12-16 cps is used.

6. Use of a delayed-release coating solution according to one of claims 1 to 5 for the preparation of diffusion coatings on pharmaceutical preparations for controlling the release of pharmaceutically active substances.

## Revendications

1. Solution d'enrobage à effet retard pour préparations pharmaceutiques contenant de l'éthylcellulose comme filmogène insoluble dans l'eau, du polyéthylèneglycol comme porogène soluble dans l'eau et comme solvant un ou plusieurs alcools à bas point d'ébullition ou des mélanges comprenant chacun un ou plusieurs alcools à bas point d'ébullition et une ou plusieurs cétones caractérisée en ce que le solvant contient une addition d'eau de 0,5 - 10% et en ce que la proportion de solides de la solution d'enrobage à effet retard elle-même contient 50 - 70% en masse d'éthylcellulose ayant une teneur en éthoxyle d'environ 45 - 50% et une viscosité (solution à 5% dans le toluène/éthanol 80:20) de 8 - 24 cps ainsi que 30 - 50% en masse de polyéthylèneglycol ayant une masse moléculaire comprise entre 4000 et 10000.

2. Solution d'enrobage à effet retard selon la revendication 1 caractérisée en ce que la proportion de solides est de 2 - 15% en masse.

3. Solution d'enrobage à effet retard selon l'une des revendications 1 et 2 caractérisée en ce que le solvant est de l'éthanol anhydre, de l'isopropanol avec une addition d'eau de 4 - 10% en masse ou un mélange d'éthanol/acétone anhydre ou d'isopropanol/acétone dans le rapport 1:1 avec une addition d'eau de 1 - 5% en masse.

4. Solution d'enrobage à effet retard selon l'une des revendications 1, 2 et 3 caractérisée en ce que l'on utilise du polyéthylèneglycol 6000.

5. Solution d'enrobage à effet retard selon l'une des revendications 1, 2, 3 et 4 caractérisée en ce que l'on utilise de l'éthylcellulose ayant une teneur en éthoxyle d'environ 48 - 49,5% et une viscosité (solution à 5% dans le toluène/éthanol 80:20) de 12 - 16 cps.

6. Utilisation d'une solution d'enrobage à effet retard selon l'une des revendications 1 à 5 pour la préparation d'enveloppes de diffusion sur des préparations pharmaceutiques pour la commande de la libération de principes actifs pharmaceutiques.
